## Europäisches Patentamt

## European Patent Office

## Office européen des brevets

(19)

(11) Numéro de publication: **0 147 283**
**B1**

(12)

# FASCICULE DE BREVET EUROPEEN

(45) Date de publication du fascicule du brevet:
16.08.89

(51) Int. Cl.⁴: **A 61 K 39/395**

(21) Numéro de dépôt: 84402531.2

(22) Date de dépôt: 07.12.84

(54) Nouveau médicament immunomodulateur, à base de fragments Fc d'IgG Humaines.

(30) Priorité: 07.12.83 FR 8319568

(43) Date de publication de la demande:
03.07.85 Bulletin 85/27

(45) Mention de la délivrance du brevet:
16.08.89 Bulletin 89/33

(84) Etats contractants désignés:
AT BE CH DE FR GB IT LI LU NL SE

(56) Documents cités:
EP-A- 0 010 309

CHEMICAL ABSTRACTS, vol. 91, no. 15, 8 octobre 1979, page 467, no. 122027p, Columbus, Ohio, US; H.E. PRINCE et al.: "Proteolysis of human IgG by human polymorphonuclear leukocyte elastase produces an Fc fragment with in vitro biological activity" & CLIN. EXP. IMMUNOL. 1979, 37(1), 162-8
CHEMICAL ABSTRACTS, vol. 94, no. 5, 2 février 1981, page 407, no. 28731d, Columbus, Ohio, US; J.H. PASSWELL et al.: "Modulation of human monocyte functions by Fc fragments of IgG: a comparison to other monocyte 'activators'" & IMMUNOLOGY 1980, 41(1), 217-25

(73) Titulaire: INSTITUT MERIEUX Société anonyme dite:, 17, rue Bourgelat, F-69002 LYON (FR)

(72) Inventeur: Carosella, Edgardo D., 27, rue Soeur Bouvier, F-69005 Lyon (FR)
Inventeur: Armand, Jacques, "Les Clos" St Germain S/Arbresle, F-69210 L'Arbresle (FR)

(74) Mandataire: Nony, Michel et al, Cabinet Nony 29, rue Cambacérès, F-75008 Paris (FR)

(56) Documents cités: (suite)
THE JOURNAL OF IMMUNOLOGY, vol. 122, no. 1, janvier 1979, pages 89-96, Williams & Wilkins Co., US; M.A. BERMAN et al.: "Lymphocyte stimulation with Fc fragments. I. Class, subclass, and domain of active fragments"

## Description

La présente invention a pour objet un nouveau médicament immunomodulateur à usage humain, à base de fragments Fc d'immunoglobulines G humaines. Ce médicament est utilisable notamment dans le traitement de dysfonctions immunitaires, y compris dans le traitement de maladies auto-immunes, et aussi comme agent inhibiteur de l'immunité de greffe, dans le traitement des malades devant subir ou ayant déjà subi une transplantation d'organe.

On sait que la pratique des greffes d'organes, notamment de reins et de moëlle, est actuellement largement répandue. Malgré les progrès dans la connaissance du système d'histocompatibilité majeure, on sait qu'il est toujours nécessaire de provoquer artificiellement une inhibition de l'immunité pour éviter la réaction de rejet par laquelle l'organisme cherche à détruire les cellules étrangères de l'organe greffé.

Pour obtenir une telle inhibition des défenses immunologiques, on utilise généralement en association diverses méthodes fondées notamment sur l'emploi de l'irradiation complète, des chimiothérapies (agents alkylants, analogues de bases puriques), de certains antibiotiques, et du sérum antilymphocytaire.

Il est bien connu que ces méthodes d'inhibition de l'immunité présentent toutes divers inconvénients; en particulier, elles paralysent non seulement les défenses naturelles de l'organisme contre la greffe, mais aussi les défenses naturelles contre les agents infectieux tels que les virus ou les microbes. Les malades se trouvent donc désarmés devant les infections, et des précautions très contraignantes doivent être prises pour les éviter.

La présente invention a pour objet un médicament qui inhibe les réactions de défense immunologique contre l'organe greffé et qui n'inhibe pas, et même au contraire augmente, les réactions de défense de l'immunité humorale dont on sait qu'elles jouent un rôle essentiel dans la lutte contre les infections.

Le principe actif du médicament de l'invention est constitué par le fragment Fc des immunoglobulines G humaines, ou ses analogues.

On sait que les immunoglobulines (en abrégé = Ig) sont des protéines qui ont été réparties selon divers critères en plusieurs classes: IgG, IgM, IgA, IgD et IgE.

Les IgG, qui sont les immunoglobulines majoritaires du sérum, ont, comme les autres immunoglobulines, une structure multicaténaire, et diverses réactions de clivage, notamment enzymatique, ont permis d'isoler plusieurs fragments de cette protéine.

C'est ainsi que par hydrolyse avec la papaïne ou la plasmine, la molécule d'IgG donne trois fragments: deux fragments possédant chacun un site anticorps, appelés fragments Fab, et un fragment n'ayant pas de site anticorps, mais ayant diverses fonctions effectrices, appelé fragment Fc.

Les méthodes d'obtention du fragment Fc ou de fragments analogues par clivage enzymatique avec la papaïne, la plasmine, ... sont bien connues et sont décrites par exemple par R.R. Porter, Biochem. J. 73, p. 119 (1959).

Sauf indication contraire, dans ce qui suit, l'expression «fragment Fc» sans autre précision désigne des fragments Fc d'IgG humaines.

Les propriétés pharmacologiques des fragments Fc, et leur rôle dans les divers mécanismes de défense immunitaire, étaient encore mal connus jusqu'à présent. Leur étude sur un modèle animal ne permet pas de tirer des conclusions sur l'activité chez d'autres espèces, car il est connu, d'une part, que les immunoglobulines, et notamment IgG, diffèrent selon les espèces, et d'autre part, qu'une immunoglobuline donnée à des actions différentes sur la réponse immunitaire de diverses espèces.

Diverses publications ont signalé que les fragments Fc d'immunoglobulines diverses ont une action stimulante sur la réponse proliférative et l'activation polyclonale des lymphocytes murins et humains, en l'absence ou en présence d'antigène. Par contre, sur les lymphocytes de souris, le fragment Fc d'immunoglobulines de chèvre n'a qu'un effet mitogène faible et le fragment Fc des immunoglobulines de lapin ou d'oie n'a aucune action stimulante, quelle que soit la dose; voir par exemple M.A. Berman et al, The Journal of Experimental Medicine, 146, pp. 241–256 (1977) et The Journal of Immunology, 122, 89–96 (1979).

On sait également que le fragment Fc de myélome humain provoque une augmentation de la prolifération des lymphocytes de la souris dans la réaction lymphocytaire mixte; voir par exemple E.L. Morgan et al. J. Exp. Med., 153, pp. 1161–1172 (1981).

On sait aussi que les fragments Fc des IgG humaines (de même que les IgG intactes) ont un effet inhibiteur de la cytotoxicité à médiation cellulaire dépendant des anticorps (ADCC); voir par exemple Wisløff et al. Scand. J. of Immunol., 3, pp. 29–38 (1974).

On sait enfin que les cellules NK («natural kiler») humaines, qui interviennent dans certaines réactions immunitaires, portent des récepteurs pour le fragment Fc des IgG, mais que l'activité cytotoxique des cellules NK n'est pas en rapport avec ce récepteur; voir M.E. Neville, Journal of Immunol., 125, p. 2604 (1980).

On a aussi déjà décrit dans la demande EP-A-10 309 l'application de mélanges de produits de clivage enzymatique d'IgG humaines contenant des Fc pour le traitement des allergies.

Compte tenu de la complexité du système de défense immunitaire, et compte tenu de la multiplicité des phénomènes de régulation de l'immunité, il faut considérer, à la lumière des connaissances actuelles dans le domaine de l'immunologie, que l'utilisation d'un produit actif comme médicament immuno-modulateur ne peut être envisagée qu'après exploration de l'ensemble des activités du produit actif sur les diverses réactions immunitaires humorales et cellulaires, et en particulier sur l'activation ou l'inhibition des cellules cytotoxiques et suppressives. Cette exploration doit en outre être nécessairement conduite sur un modèle isologue, dans lequel il est absolument impossible d'envisager la moindre application comme médicament, pour les raisons qui ont été évoquées ci-dessus.

On a maintenant découvert, par l'étude des propriétés du fragment Fc des IgG humaines sur un modèle isologue, des propriétés pharmacologiques intéressantes qui permettent de préconiser l'utilisation de ce fragment comme médicament immunomodulateur permettant notamment de traiter les maladies auto-immunes et permettant aussi d'éviter les réactions de rejet des greffes d'organes, ou d'en diminuer les effets.

Les fragments Fc des IgG humaines, stimulants de la réponse proliférative lymphocytaire, n'ont pas d'action sur la réaction allogénique primaire. Par contre, les fragments Fab et les IgG intactes ont une action inhibitrice sur la réaction allogénique primaire et n'ont pas d'action sur la réponse proliférative.

Le fragment Fc des IgG humaines, tout comme le fragment Fab et l'IgG intacte, n'a ni action stimulante ni action inhibitrice sur la réponse lymphocytaire en présence d'antigène.

Les fragments Fc des IgG humaines n'ont pas d'action inhibitrice ni stimulatrice du phénomène de génération de cellules cytotoxiques dans la réaction lymphocytaire mixte primaire (CML I). Toutefois, on a découvert que les fragments Fc ont une activité inhibitrice de la cytotoxicité dans la réaction lymphocytaire mixte secondaire (CML II), comme l'IgG intacte et le fragment Fab.

La découverte de cette dernière propriété a conduit à étudier l'influence éventuelle du fragment Fc sur la stimulation ou la prolifération de cellules suppressives, et les études reproduites ci-après dans la partie expérimentale montrent que les fragments Fc d'IgG humaines ont une activité favorisant le développement des lymphocytes suppresseurs ainsi qu'une activité adjuvante de la stimulation des cellules pré-suppressives.

Enfin, l'étude de l'influence des fragments Fc sur la production des immunoglobulines totales dans une réaction de type mitogénique a permis de montrer une augmentation de la production des immunoglobulines totales, alors que le Fab est inactif et que les IgG intactes conduisent au contraire à une inhibition de la production des immunoglobulines totales; voir A. Durandy et al, J. Clin. Invest., 67, pp. 867–877 (Mars 1981).

Il convient de noter que dans l'inhibition de l'activité cytotoxique anticorps – dépendante (ADCC) et dans la différenciation des cellules suppressives, on observe un effet proportionnel à la dose de produit étudié. Cela permet de penser que le fragment Fc agit directement sur certaines sous-populations lymphocytaires T.

Ces diverses propriétés des fragments Fc des IgG humaines permettent donc de les utiliser comme des médicaments immunomodulateurs qui inhibent l'immunité cellulaire tout en favorisant l'immunité humorale, et qui sont donc avantageusement utilisables dans le traitement des malades recevant des greffes d'organes ou souffrant de dysfonctions immunitaires, et notamment de maladies auto-immunes.

Le médicament de l'invention est caractérisé par le fait que son principe actif est constitué par des fragments Fc d'IgG humaines, ou par des analogues desdits fragments, et qu'il est exempt d'IgG intactes et/ou de fragments Fab ou qu'il n'en contient pas plus de 2% en poids par rapport au poids total (Fc + Fab + IgG).

Dans la présente demande on désigne par l'expression «analogues des fragments Fc d'IgG humaines» tous fragments ou sous-fragments de Fc, et tous peptides synthétiques ou semi-synthétiques contenant des séquences peptidiques présentes sur le fragment Fc, ainsi que tous dérivés de ces fragments, sous-fragments ou peptides, à condition que ces fragments, sous-fragments, peptides ou dérivés satisfassent aux conditions suivantes:
– ils ont des propriétés d'activateurs de la différenciation des cellules suppressives;
– ils ont des propriétés d'inhibiteurs de la cytotoxicité anticorps-dépendante;
– ils n'ont pas de propriétés d'activateurs des cellules NK.

Le médicament de l'invention peut être administré par voie orale, intraveineuse ou intramusculaires. A cet effet, il est présenté notamment sous la forme de capsules, de gélules, de dragées, de solutions injectables ou de poudre lyophilisée pour solutions injectables.

Pour l'administration par voie orale, la forme pharmaceutique doit comporter un enrobage acido-résistant permettant le passage de la barrière stomacale.

Dans le cas d'une transplantation d'organe, l'administration du médicament de l'invention peut être effectuée selon des modalités diverses en fonction des techniques de dépression immunitaire utilisées par le médecin avant et après la greffe.

Le médicament de l'invention peut être administré avant la transplantation d'organe pour provoquer la dépression de l'immunité de greffe, ou après la transplantation, en relais ou avec d'autres médications suppressives de l'immunité; il faut tenir compte du fait qu'avec le médicament de l'invention, l'effet immunomodulateur n'est obtenu généralement qu'au bout de 6 à 10 jours.

La posologie dépend notamment du mode d'administration et de la phase du traitement. Elle varie généralement de 1 mg à 10 g de principe actif par jour chez l'adulte. Par exemple, par voie intraveineuse, pour le fragment Fc, elle peut varier de 1 g à 10 g par jour chez l'adulte.

L'invention a également pour objet l'utilisation du fragment Fc des IgG humaines ou de ses analogues, dans la préparation d'un médicament immunomodulateur capable notamment de traiter les maladies auto-immunes et de prévenir les réactions de rejet, ou d'en diminuer les effets, chez les malades devant subir ou ayant subi une greffe d'organe; cette utilisation comprend notamment la mise sous une forme pharmaceutiquement convenable, selon les méthodes usuelles.

Bien entendu, le médicament de l'invention peut être utilisé en association avec d'autres médicaments immunodépresseurs ou corticoïdes.

Les exemples suivants illustrent l'invention sans toutefois la limiter.

Dans ces exemples, on a utilisé les diverses abréviations suivantes:

- ADCC: Cytotoxicité à médiation cellulaire dépendant des anticorps;
- BSA: Sérum albumine bovine;
- CML: Cytotoxicité à médiation lymphocytaire;
- F(ab')2: Fragment obtenu par clivage des IgG avec la pepsine;
- GRP: Globules rouges de poulet;
- Haplotype DR: Déterminant «D-related» du système HLA;
- HLA Dr, HLA A, B, HLA D: Déterminants antigéniques du système d'histocompatibilité leucocytaire humain;
- Ig: Immunoglobuline;
- MLR: Réaction lymphocytaire mixte;
- PBS: Solution de tampon phosphate;
- PHA-M: Phytohémagglutinine-M;
- PWM (pokeweed mitogen): Mitogène de phytolaque;
- SVF: Sérum de veau fœtal;
- SrAB: Sérum AB.

Dans les exemples suivants, les fragments Fc ont été séparés des fragments Fab, après clivage des IgG à la plasmine, par effet de relargage salin (à l'aide de sulfate d'ammonium) suivi de tamisage moléculaire sur colonne (gelfiltration).

EXEMPLE 1
ETUDE DES PROPRIÉTÉS PHARMACOLOGIQUES DU FRAGMENT Fc DES IMMUNOGLOBULINES HUMAINES
I – ETUDE DE LA PROLIFÉRATION LYMPHOCYTAIRE
Recherche d'une activité proliférative lymphocytaire des fragments Fc et Fab des IgG et de l'IgG.

MATÉRIEL ET MÉTHODE
1. Préparation des différentes populations lymphocytaires
a. Obtention des lymphocytes
Les lymphocytes de sang veineux périphérique de 60 donneurs volontaires de sang ont été séparés sur un gradient de Ficoll-radioselectan (d: 1077) suivant la technique de Böyum A. (Scand. J. Clin. Lab. Invest. Suppl. 97, 1968, 21, p. 1).

b. Obtention d'une population lymphocytaire enrichie en macrophages
La séparation des monocytes d'une population lymphocytaire précédemment obtenue a été effectuée sur un gradient de Percoll (d: 1064) suivant la technique d'Ulmer and Flad (J. Immunol. Methods, 1979, 30, p. 1).

c. Obtention des lymphocytes déplétés en cellules adhérentes
La population de lymphocytes obtenue précédemment (a) a été incubée pendant 1 heure à 37 °C dans un flacon (type Falcon); des cellules non adhérentes ont été récupérées.

Ces trois types (a, b, c) de populations cellulaires ont été conservés à −260 °C et décongelés au moment du test.

d. Réaction de peroxydase
Le pourcentage des monocytes dans chaque population lymphocytaire a été déterminé par la réaction de peroxydase combinée d'après SATO (Traité de biologie appliquée, 1964, vol. 3, p. 95) sur une numération totale de 400 cellules par frottis pour chaque individu.

2. Fragments Fc-Fab et IgG
Les préparations des différents sous-fragments ont été effectuées selon les méthodes connues au départ d'une IgG humaine plasmatique traitée à la plasmine. Les IgG (PRG) sont de type $\gamma 1$ (30%) et $\gamma 2$ (70%). Le Fab présente une contamination de 4,1% de Fc, par contre le Fc est pur.

3. Mise en culture
a. Le test de transformation lymphocytaire a été réalisé sur des microplaques à fond rond (96 godets par plaque) avec du RPMI additionné de 20% de sérum autologue décomplémenté.

Dans chaque godet sont réparties 200 000 cellules sous 150 µml de RMPI 20% de sérum autologue et 50 µl de Fc ou Fab ou IgG ou PWM (Gibco), dilués en RPMI. Chaque dilution de chaque fragment a été testée par triplicate.

b. Incubation: Les plaques ont été incubées 6 jours dans une étuve à 37 °C, saturée d'humidité, sous une atmosphère de 5% $CO_2$ pour 95% d'air.

c. Marquage à la thymidine tritiée: à la 144ème heure, on a ajouté dans chaque puits 1 microcurie de thymidine tritiée. L'arrêt de l'incorporation se fait après 16 heures à 18 heures par refroidissement à +4 °C.

d. Précipitation: Les cellules ont été recueillies par précipitation sur skatron et comptées à l'aide d'une solution scintillante dans un compteur Béta.

RESULTATS
Sur 60 individus testés, 43,4% ont été des hauts répondeurs ($\geq$ 10 000 cpm), 18,3% des bas répondeurs (< 10 000 cpm) et pour 38,3% il y a eu absence de réponse ($\leq$ 2000 cpm).

Le critère de haut ou de bas répondeur a été établi suivant la réponse proliférative pour la culture cellulaire avec le fragment Fc (Tableau 1).

Le pourcentage de cellules peroxydase positives a été dans les trois groupes de 32%. Par contre, dans la population de lymphocytes déplétés en cellules adhérentes, il était de 1 à 2%.

Les fragments Fc, Fab et l'IgG ont été testés à 6,12 et 24 µg par culture; 50 et 100 µg ne donnent pas de différences significatives avec le groupe précédent (D–N–S).

Les résultats sont résumés dans le tableau 1.

CONCLUSIONS

Ces essais mettent en évidence l'action stimulante du fragment Fc dans la réponse proliférative lymphocytaire, en comparaison au fragment Fab ou à l'IgG entière, ainsi que la présence indispensable des macrophages nécessaire pour cette activité. Ces résultats sont à rapprocher des travaux réalisés par Weigle sur les lymphocytes de souris (J. Exp. Med. 1977, 146, p. 241) où la réponse proliférative maximale est à 5 jours et à 10 µg par culture. De même, la déplétion en cellules adhérentes entraîne une absence de réponse proliférative des lymphocytes de souris (J. Exp. Med. 1979, 150, p. 256).

II. ETUDE DE LA PROLIFÉRATION LYMPHOCYTAIRE ALLOGÉNIQUE

Recherche d'une activité adjuvante ou inhibitrice des fragments FC et Fab des IgG ainsi que des IgG intactes dans la réponse proliférative allogénique primaire.

Matériel et méthode

Les techniques de séparation des lymphocytes, des monocytes et d'obtention des différents fragments ont été décrites ci-dessus.

– Obtention des lymphocytes T

La séparation des lymphocytes T a été effectuée sur une colonne de laine de nylon (d'après la technique de Julius M. et al., Eur. J. Immunol., 1973, 3, p. 645). 40 millions de lymphocytes ont été incubés pendant 30 mm à 37 °C avec 0,6 g de laine de nylon dans une seringue de 10 ml.

Les cellules non adhérentes ont été récupérées. Des contrôles de coloration de peroxydases et rosettes E ont été faits pour bien confirmer l'enrichissement en lymphocytes T.

Ces cellules ont été utilisées comme cellules répondantes de la culture mixte.

– Récupération des surnageants de cultures de lymphocytes en présence des différents fragments de l'Ig, (Vol. 128, p. 590)

Suivant la technique de Marylin Thoman (J. Immunol., 1982) des lymphocytes enrichis en monocytes (1 × 10⁶/ml) ont été incubés pendant 24 h à 37 °C en milieu RPMI 1640 − 20% SrAB avec 60 µg de Fc, Fab ou Ig. Après 24 heures, les surnageants ont été récupérés par centrifugation.

– Culture mixte de lymphocytes

Les cellules répondantes (cellules non adhérentes) ont été dénombrées et ajustées à la concentration de 1 × 10⁶/ml. Les cellules stimulantes (population lymphocytaire totale ou lymphocytes enrichis en monocytes) ont été traitées avec 25 µg de mitomycine C par ml et par million de cellules (d'après la technique de BACH, Science, 1966, Vol. 153, p. 545) pendant 30 mm à 37 °C.

Après traitement, les cellules ont été lavées deux fois avec RPMI 10% SrAB. La suspension cellulaire a été ajustée à différentes concentrations: 1 × 10⁶/ml, 12 × 10⁴/ml, 6 × 10⁴/ml et 3 × 10⁴/ml.

La distribution de cellules stimulantes et répondantes a été faite dans des microplaques à fond conique à l'aide d'une seringue Hamilton, à raison de: 0,05 ml de cellules répondantes, 0,05 ml de cellules stimulantes, 0,05 ml de RPMI, 20% SrAB.

Les microplaques ont été incubées 5 jours à l'étuve à 37 °C −5% CO2. A la 96ème heure, on a ajouté 2 µCu de thymidine tritiée par godet pendant 18 heures. Les cultures ont été recueillies par précipitation sur des filtres FLOW à l'aide d'un microprécipitateur Skatron. Chaque filtre après séchage a été placé dans un tube plastique; 3 ml de solution scintillante ont été ajoutés dans chaque tube et ensuite mis à compter dans un compteur à scintillation liquide. Les résultats ont été exprimés en coups par minute (cpm).

– Résultats
– MLR et Fc, Fab et Ig à différentes concentrations

Les différents fragments: Fc, Fab, Ig à différentes concentrations ont été ajoutés à une MLR I.

Le fragment Fc à différentes concentrations (12, 25, 50 et 100 µg/culture) n'a pas montré d'action adjuvante ou inhibitrice de la MLR I, en comparaison avec RPMI.

Par contre, le fragment Fab à la concentration de 100 µg/culture montrait une inhibition de la MLR I. Les IgG aux concentrations de 50 et 100 µg entraînaient une inhibition de la MLR I.

– Fc, Fab et IgG sur une MLR avec différentes concentrations de cellules stimulantes

Cette technique a été effectuée pour mettre en évidence l'action des différents fragments dans des conditions sous-optimales de la réponse proliférative. Aucune différence significative en comparaison avec le RPMI n'a été observée; seule l'IgG à la concentration de 50 µg donnait une inhibition de la MLR I.

– Fc, Fab et Ig sur une MLR avec des cellules stimulantes enrichies en monocytes

Etant donné que l'activation des lymphocytes T par l'induction du fragment Fc se fait à travers les macrophages, on a essayé de se placer dans un système plus propice, en conservant les conditions sous-optimales de culture. Aucun effet adjuvant aux différentes doses testées n'a été observé. Un effet inhibiteur a été remarqué pour l'IgG à 24 µg/culture.

– MLR et surnageants de cellules incubées avec Fc, Fab ou Ig.

Comme la réponse proliférative se déclenche dans les premières heures qui suivent le contact entre cellules stimulantes et répondantes et comme la production de sous-fragments Fc par les macrophages se fait pendant les 24 premières heures, on a ajouté dans une MLR les surnageants provenant des lymphocytes enrichis en monocytes, incubés pendant 24 h à 37 °C avec les différents fragments.

Ces surnageants ont été préparés à partir de cellules autologues de la cellule répondante et testés dans une MLR I dans des conditions optimales et sous-optimales de prolifération.

Aucun effet inhibiteur ni adjuvant n'a été observé avec les fragments Fc, Fab ou Ig.

Conclusion

Avec le fragment Fc, aucune activité significative adjuvante ou inhibitrice n'a été observée, pour les lymphocytes humains, aux concentrations de 6, 12, 24, 50 et 100 µg pour 50 000 cellules dans la réaction lymphocytaire allogénique primaire. Le choix des doses employées a été déterminé lors de la mise en évidence de l'action proliférative du fragment Fc sur les lymphocytes (protocole I ci-dessus).

Par contre, l'immunoglobuline entière et le fragment Fab donnent une inhibition de la réponse allogénique primaire dans tous les modèles testés.

III – ETUDE DE L'ACTIVITÉ SUR LES LYMPHOCYTES CYTOTOXIQUES (CML I ET CML II)

Exploration de l'activité des fragments Fc, Fab et de l'IgG dans les différents types de cytotoxicités.

Matériel et méthodes

Les techniques de séparation de lymphocytes de sang périphérique de donneurs volontaires et l'obtention des différents fragments ont été décrites ci-dessus.

Fc, Fab et IgG
Gamma placentaire digérée à la plasmine.

| IgG sous-classes: | |
|---|---|
| IgG1 | = 57% |
| IgG2 | = 39% |
| IgG3 | = 0,2% |
| IgG4 | = 3,7% |

Cytotoxicité anticorps dépendante à médiation cellulaire (ADCC).

La technique utilisée est celle décrite par F. Weisløff, T.E. Michaelsen, S.S. Frøland, Scand. J. Immunol., 3, pp. 29–38, 1974.

Les cellules cibles sont des GRP sensibilisés avec des IgG de lapin anti-GRP et marqués au Chrome 51.

Test ADCC

Les cellules effectrices (lymphocytes de sang périphérique) ont été dénombrées et ajustées à une concentration de $5 \times 10^6$/ml en RPMI 10% SVF.

A l'aide d'une seringue Hamilton, on a distribué dans les microplaques à fond rond 100 µl de cellules effectrices, 50 µl des produits à tester à différentes dilutions par triplicate.

Après une incubation de 20 min à 37 °C, 50 µl de GRP sensibilisés et marqués ont été distribués, donnant un rapport final de 50 cellules effectrices contre une cellule cible (50:1).

Le relargage spontané a été déterminé en incubant les GRP avec du milieu RPMI 10% SVF, le relargage maximum en ajoutant aux GRP de l'acide chlorhydrique 1 N. Comme témoin de l'activité de l'antisérum, les cellules effectrices ont été mises en face de GRP préincubés avec du sérum de lapin normal.

Les plaques, après une incubation de 18 h à 37° sous 5% $CO_2$ en atmosphère humide, ont été centrifugées 10 min. à 800 rpm et la récupération de surnageant a été effectuée sur des tampons filtres (Flow, Ref. 78-21-005).

Cytotoxicité «Natural Killer» (NK)

La technique utilisée est celle décrite par B.M. Vose, F. Wanky, S. Argov et E. Klein, Eur. J. Immunol. 1977, 7, pp. 753–757.

Les cellules cibles sont des cellules K 562 (lignée myéloide humaine) marquées au chrome 51.

Test NK

Les cellules effectrices (lymphocytes de sang périphérique) ont été ajustées à une concentration de $5 \cdot 10^6$/ml, $2,5 \cdot 10^6$/ml, $1,2 \cdot 10^6$/ml et $6 \cdot 10^5$/ml.

Leur distribution et leur traitement ont suivi la même méthodologie que pour la technique ADCC. Les cellules cibles ont été distribuées sous 50 µl donnant un rapport final de 50 cellules effectrices contre une cible (50:1, 25:1, 12:1 et 6:1). Le relargage spontané ainsi que le relargage maximum, et la récupération des surnageants sont effectués dans les mêmes conditions que dans le test ADCC.

Cytotoxicité à médiation lymphocytaire primaire I (CML I) et secondaire (CML II)

Cette étude est effectuée selon la méthode de Lightbody et al, Gen. Bact. Virol. Immunol., 1971, Vol. 64, p. 243.

Cellules effectrices

La phase de sensibilisation allogénique correspond à une culture mixte identique à celle précédemment décrite (voir II ci-dessus).

Dans les deux tests, la sensibilisation s'est effectuée contre un seul haplotype D r en tenant compte de la différence entre HLA A, B des cellules répondantes et stimulantes.

Cellules cibles

Dans les deux tests on a utilisé comme cellules cibles la cellule stimulante qui a servi à la sensibilisation des MLR respectives. Ces cellules stimulées pendant 72 h par la PHA-M (Gibco) ont été marquées par du chrome 51 18 heures avant le test, à raison de 200 µl pour $10 \cdot 10^6$ cellules.

Après des lavages successifs, les cellules ont été dénombrées au bleu trypan et ajustées à $200 \cdot 10^3$/ml. Le test de CML est alors réalisé dans des microplaques à fond rond. Les cellules effectrices sous 100 µl, traitées comme il a été décrit précédemment, sont incubées avec 50 µl de cellules cibles pendant 4 heures à 37 °C sous 5% $CO_2$ en atmosphère humide, donnant un rapport de 50:1 dans le cas de la CML I et de 50:1, 25:1, 12:1, 6:1 dans le cas de la CML II. Le relargage spontané ainsi que le relargage maximum et la récupération des surnageants sont effectués dans les conditions décrites précédemment. Dans les quatre tests de cytotoxicité, le relargage de chrome 51 a été mesuré sur un compteur gamma pendant 2 min. Les résultats ont été exprimés en pourcentage de cytotoxicité suivant la formule:

$$\text{Pourcentage de relargage de Cr 51} = \left( \frac{\text{cpm relargage} - \text{cpm relargage spontané}}{\text{cpm relargage maximum} - \text{cpm relargage spontané}} \right) \times 100$$

On considère comme positive toute cytotoxicité supérieure à 10%.

RESULTATS

Cytotoxicité anticorps dépendant

Une inhibition de la cytotoxicité anticorps dépendant avec le fragment Fc et l'Ig a été observée chez tous les individus testés. Cette inhibition augmente de façon proportionnellement directe avec la concentration de fragment Fc ou d'IgG (tableau 2).

L'inhibition de l'activité Killer s'est révélée toujours plus importante avec le fragment Fc qu'avec les IgG; c'est ainsi que 6 µg de Fc donnent une inhibition égale à celle de l'Ig à 50 µg.

Par contre, aucun effet inhibiteur n'a été observé avec le fragment Fab, même à des doses très élevées.

Activité «Natural Killer»

Aucune inhibition de l'activité NK n'a pu être mise en évidence avec les fragments Fc-Fab ou l'Ig, même à des doses très importantes. Pour confirmer ces résultats et à deux doses extrêmes (6 µg et 100 µg) l'activité NK a été testée dans différents rapports cellules effectrices-cellules cibles. Aucune différence significative, à aucune dose ni à aucun rapport, n'a été observée (Tableau 3).

Cytotoxicité à médiation cellulaire primaire et secondaire.

La CML I n'a pas été modifiée par l'action des fragments Fc, Fab et de l'IgG testés à différentes doses.

Par contre, la CML II a été inhibée par les fragments Fc et Fab à la dose de 25, 50 et 100 µg; les IgG ont montré une inhibition significative à toutes les doses employées (tableau 4).

V. ETUDE DE L'ACTION DES FRAGMENTS Fc-Fab ET Ig HUMAINES SUR LES ACTIVITÉS DE LA CELLULE SUPPRESSIVE ET PRÉSUPPRESSIVE DE LA RÉPONSE ALLOGÉNIQUE PRIMAIRE.

Objet

Exploration de l'activité des fragments Fc, Fab et de l'Ig sur des cellules T suppressives de la réponse proliférative allogénique et dans la différenciation des cellules présuppressives.

Matériel et méthode

Les techniques de séparation des lymphocytes du sang périphérique de donneurs volontaires et l'obtention des différents fragments ont été décrites ci-dessus.

Gamma placentaire digérée à la plasmine: pureté des fragments utilisés par gel filtration ACA 44: Fc: 100%, Fab: 94,6%, (5,4% 7S), Ig (PRG) 7S: 95,4% 10S: 2,8% 10S: 1,8%, IgG1: 57%, IgG2: 39%, IgG3: 0,2%, IgG4: 3,7%.

Les donneurs volontaires du sang ont été étudiés pour les principaux déterminants du complexe HLA-Dr, par sérologie et lymphotoxicité classique ou modifiée (suspension lymphocytaire enrichie en cellules B).

Allosensibilisation: M.J. Sheeny, F.H. Bach, Tissue Antigens, 1976, 8, pp. 157–171.

Pour la sensibilisation allogénique in vitro, $10 \cdot 10^6$ lymphocytes utilisés comme répondeurs sont mis en présence de $10 \cdot 10^6$ cellules allogéniques mitomycinées, incompatibles pour un Ag HLA-Dr ($25\,\mu g/10^6$ cellules/ml), utilisées comme stimulateurs. La culture est réalisée dans des bouteilles Falcon (CA 3013) avec 20 ml de milieu RPMI 1640 additionné de 10% d'un pool de sérum AB dans une étuve humidifiée à 37°C, en présence de 5% de $CO_2$, pour une période de 10 jours.

Test de Suppression (J. Dausset, Nature, 1978, 267, pp. 502).

L'activité suppressive des lymphocytes allosensibilisés in vitro a été évaluée dans une MLR I avec présence de trois populations de cellules: les lymphocytes répondeurs, les cellules stimulantes mitomycinées et les cellules allosensibilisées également mitomycinées (ces cellules sont autologues aux cellules répondantes).

Le comptage est effectué dans un compteur à scintillation liquide.

Le pourcentage de suppression est évalué selon la formule:

$$\% \text{ suppression} = \left( 1 - \frac{\text{Valeur médiane expérimentale (CPM)}}{\text{Valeur médiane témoin (CPM)}} \right) \times 100$$

Etude de l'activité des différents fragments sur les cellules, pré-suppressives et suppressives Pour mettre en évidence une action stimulante des cellules pré-suppressives, deux modèles différents ont été essayés.

Le premier consiste à la pré-incubation pendant une heure des lymphocytes totaux de sang périphérique avec les différents fragments de Fc, Fab ou IgG. Après plusieurs lavages, ces cellules ont été mitomycinées et mises comme troisièmes cellules dans une MLR I dans les conditions précédemment décrites. Le groupe témoin a été pré-incubé avec RPMI.

Le deuxième protocole permet de mettre en évidence un effet adjuvant éventuel sur le développement des cellules suppressives. Dans l'étape d'allosensibilisation, les fragments Fc, Fab et IgG ont été ajoutés au jour 0 à différentes doses. Après 10 jours de sensibilisation, ces cellules sont ajoutées comme troisièmes cellules dans une MLR I dans les conditions précédemment décrites.

Enfin, pour décrire l'activité de ces fragments sur les cellules suppressives, différentes doses de Fc, Fab, Ig ont été ajoutées dans le test à trois cellules.

Résultats (tableau 5)

1. Activité des fragments Fc, Fab et Ig dans la pré-incubation de cellules non sensibilisées et ajoutées comme troisièmes cellules Lors du test de suppression, différentes doses de fragments Fc ou Fab, ou d'Ig ont été incubées pendant une heure avec les cellules sensibilisées, avant d'être lavées et mitomycinées.

Les cellules préalablement incubées avec le fragment Fc donnent une augmentation de l'inhibition de la MLR I proportionnelle à la dose de Fc utilisée. Cette augmentation est significative pour les doses de 15 et 30 µg/ml. On retrouve une inhibition significative de la MLR avec les cellules pré-incubées en présence de l'Ig à la dose de 15 µg.

Par contre, les cellules pré-incubées avec le fragment Fab ne révèlent pas d'inhibition significative de la réponse allogénique.

### 2. Activité du fragment Fc, Fab, IgG dans l'allosensibilisation

Différentes doses de fragments Fc, Fab et IgG intacte ont été ajoutées au jour 0 de l'allosensibilisation.

Les cellules suppressives sensibilisées en présence du fragment Fc montrent une activité inhibitrice de la MLR I significativement plus élevée que les cellules témoins aussi bien à 1,5 µg qu'à 100 µg par culture. D'autre part, les cellules incubées avec l'Ig intacte montrent une augmentation significative de l'inhibition de la MLR I, à des doses supérieures à 3 µg. Par contre, les cellules sensibilisées en présence du fragment Fab, ne donnent aucune différence significative.

Dans le cas où le pourcentage de suppression a été très élevé, il est par conséquent difficile d'évaluer l'augmentation. Une expérience supplémentaire a été réalisée. Les cellules allosensibilisées et mitomycinées ont été ajoutées à différentes concentrations donnant un rapport de: 1:1, 2:1, 4:1, cellules répondantes/cellules suppressives. Dans ce cas-là, on retrouve une augmentation significative de l'activité suppressive à la dilution 8:1 sur les cellules qui ont été sensibilisées en présence du fragment Fc.

### 3. Activité des fragments Fc, Fab, Ig dans le test de suppression de la réponse allogénique I

Différentes doses de fragments Fc, Fab, Ig ont été introduites dans la MLR I à trois cellules au moment du test. Aucune activité adjuvante ni inhibitrice n'a été observée.

Conclusions:

La première indication de l'existence de cellules suppressives développées au cours de la réponse secondaire est venue de l'observation que des cellules allosensibilisées deux fois contre le même alloantigène donnaient une réponse proliférative moindre que celle obtenue dans le cas d'une seule allosensibilisation et la démonstration d'un processus actif a été apportée par l'addition dans une MLR classique primaire de ces cellules allosensibilisées, irradiées.

Les cellules suppressives sont spécifiques de l'antigène HLA-Dr présenté par la cellule stimulante. Elles apparaissent comme des lymphocytes T, puisqu'au bout de 10 jours de culture, 95% des cellules allosensibilisées forment de rosettes E, moins de 3% ont des IgG de surface et moins de 2% des monocytes. Le mécanisme de suppression provoqué par une hyperimmunisation allogénique in vitro paraît très proche du processus multitransfusionnel pratiqué avant une greffe rénale, lequel aboutit à une survie améliorée de la greffe, comme il a été rapporté par Opelz et Teroski (Transplantation, 1980, 29, p. 153).

Les expériences précédemment décrites ont eu comme objectif la mise en évidence de l'activité des fragments Fc, Fab et des IgG dans le développement des lymphocytes pré-suppresseurs, ainsi que l'activité directe sur les cellules suppressives.

## V. ETUDE DE LA PRODUCTION DES IMMUNOGLOBULINES TOTALES A PARTIR DES FRAGMENTS Fc, Fab, IgG

Objectif: Mise en évidence de la production des immunoglobulines après stimulation par le Fc, Fab et IgG.

Matériel et méthodes

1. Préparation des différentes populations lymphocytaires.

Techniques de séparation de lymphocytes du sang périphérique de donneurs volontaires et l'obtention des différentes sous-populations, comme l'obtention des différents fragments: voir ci-dessus. Gamma placentaire digérée à la plasmine: (pureté et caractéristiques: voir ci-dessus).

2. Marquage à l'iode 125 de l'antisérum

Du F (ab')2 de chèvre anti-Ig totales humaines (laboratoires Cappel) est marqué avec l'iode 125 S4.

Ensuite, le mélange de F (ab')2 marqué et d'iode en excès est déposé sur une colonne Sephadex G 50. Le premier pic correspond au F (ab')2 (PM 50 000), le deuxième pic correspond à un volume de 8 à 10 ml suivant les résultats du marquage en tampon phosphate 0,5 M pH 7,5 et comme support protéique 1% final de sérum de cheval sans gammaglobuline est ajouté.

Le rendement de marquage est calculé suivant la formule:

$$\frac{\text{cpm pic F (ab')2}}{\text{cpm pic F (ab')2} + \text{cpm I}_{125} \text{ en excès}} \times 100$$

L'activité spécifique se calcule comme suit:

$$\frac{\text{cpm}}{\text{ng F (ab')2 marqué}}$$

$$\text{ng F (ab')2 marqué} =$$

$$1000 \times \frac{\text{vol. prise essai (µl)} \times \text{qté F(ab')2(µg)}}{\text{vol. final de F(ab')2 marqué (µl)}}$$

3. Titrage des IgG humaines: test RIA

Après une nuit à +4°C, d'absorption du F (ab')2 de chèvre anti-Ig humaines dans des cupules interchangeables, on procède à l'aspiration et à la saturation de ces cupules avec du PBS +5% BSA pendant une heure à 37°C.

A. Incubation des produits à tester
– Dilution de la gamma globuline étalon.
Comme étalon, on a utilisé de la gamma globuline placentaire à une concentration de 60 µg/ml.

On effectue dix dilutions successives de 1/2 en 1/2 de cette solution, en PBS plus 0,5% BSA, plus 0,05 Tween 20.

– Dilution de produits à tester.

Après avoir mis en culture pendant 6 jours des cellules de sang périphérique, des cellules non adhérentes et des cellules enrichies en macrophages, à 1,10⁶/ml en RPMI plus 10% SVF, en présence soit de FC, Fab, Ig ou RPMI, à raison de 60 µg/ml, les surnageants sont recueillis après centrifugation.

Ils sont ensuite utilisés à part, 1/5, 1/25 et 1/125 en PBS 0,5% BSA plus 0,05% Tween 20.

La distribution de chaque dilution de produit se fait à raison de 100 µl par cupule en triplicate.

Celles-ci sont ensuite incubées 2 h à 37 °C, et rincées plusieurs fois.

B. Contact antigène – F (ab')2 chèvre anti Ig humaines marqué à l'iode 125

100 µl de F (ab')2 marqué et dilué sont distribués dans chaque cupule. Après 2 h d'incubation à 37 °C, plusieurs lavages sont faits avec du PBS Tween. Suit une aspiration complète de chaque cupule. Le comptage s'effectue sur un compteur gamma.

C. Détermination de la quantité d'Ig dans les surnageants

cpm réel = cpm (Fc* + lympho + RPMI sérum) – cpm (FC + RPMI sérum)

* Fc ou Fab ou Ig.

Ensuite, ces cpm sont analysés sur une étude de régression de courbe en fonction de l'étalon.

RÉSULTATS ET CONCLUSIONS (Tableau 6)

La production des immunoglobulines totales à partir de différentes sous-populations lymphocytaires, en présence de fragment Fc s'est montré significativement plus élevée qu'avec le fragment Fab.

Par contre, les IgG apparaissent plutôt inhibitrices de production des IgG totales que stimulatrices, en comparaison avec la production spontanée des IgG.

L'introduction des immunoglobulines et des sous-fragments dans la technique de RIA oblige à faire des contrôles parallèles sur des surnageants 5 jours sans cellules pour obtenir des résultats comparables.

Cette méthodologie aide à mettre en évidence la production in vitro des IgG à partir de lymphocytes stimulés avec le Fc.

EXEMPLE 2
RÉALISATION D'UNE COMPOSITION PHARMACEUTIQUE

Les fragments Fc sont préparés à partir d'IgG humaines purifiées par la technique d'Oncley J.L. et coll., J. of Am. Chem. Soc., 1949, 71, p. 541, et traitées par la plasmine humaine selon la technique de Plan R. et Makula M.F., Vox Sanguinis, 1975, Vol. 28, pp. 157–175. Ces IgG proviennent de sang prélevé à la veine ou exprimé de placentas. La solution de fragments Fc apyrogène est filtrée stérilement, répartie à raison de 2,5 g de principe actif par flacon et lyophilisée.

Au moment de l'utilisation, la solution est reconstituée avec de l'eau employée pour préparations injectables.

Tableau 1

| µg par culture | N° | Moyenne ± ES CPM incorporation H3 | | | | | Répondeurs |
|---|---|---|---|---|---|---|---|
| | | Fc | Fab | IgG | RPMI | PWM | |
| 6 | 26 | 23308 ± 4507 | 11446 ± 1665 | 10985 ± 1440 | 6930 ± 569 | 66884 ± 4322 | Hauts répondeurs 43,4% |
| 12 | 26 | 18324 ± 2026 | 13641 ± 2339 | 13715 ± 2199 | 6930 ± 569 | 66884 ± 4322 | |
| 24 | 26 | 21309 ± 2630 | 15410 ± 5093 | 10024 ± 1463 | 6930 ± 569 | 66884 ± 4322 | |
| 6 | 11 | 6161 ± 400 | 8763 ± 631 | 3010 ± 489 | 4100 ± 508 | 47319 ± 6898 | Bas répondeurs 18,3% |
| 12 | 11 | 7278 ± 948 | 3985 ± 1298 | 3126 ± 593 | 4100 ± 508 | 47319 ± 6898 | |
| 24 | 11 | 8032 ± 671 | 5248 ± 1179 | 5128 ± 567 | 4100 ± 508 | 47319 ± 6898 | |
| 6 | 9 | 1385 ± 413 | 1110 ± 349 | 1483 ± 433 | 1689 ± 334 | 37145 ± 4717 | Cellules adhérente (−) |
| 12 | 9 | 1369 ± 471 | 1137 ± 288 | 1064 ± 334 | 1689 ± 334 | 37145 ± 4717 | |
| 24 | 9 | 1435 ± 489 | 1109 ± 244 | 1661 ± 423 | 1689 ± 334 | 37145 ± 4717 | |

Sérum autologue          peroxydase 32%

Tableau 2
ADCC et fragment Fc, Fab, IgG – Test ADCC 50:1

| Prod dose puits | % cytotoxicité RE1 Cr 51 M ± ES n = 7 | | | |
|---|---|---|---|---|
| | Fc | Fab | IgG | RPMI (témoin) |
| ** 6 µg | 25,2 ± 5,5 >46%* (a) | 46,8 ± 8,2 >0,01 | 33,1 ± 5,8 >30%* | 47,4 ± 7,4 (h) |
| 12 µg | 19,1 ± 4,56 >59% (b) | 47,9 ± 7,6 0 | 30,6 ± 5,5 >35* (e) | 47,4 ± 7,4 |

Tableau 2 (Suite page suivante)
ADCC et fragment Fc, Fab, IgG – Test ADCC 50:1

| Prod dose puits | % cytotoxicité RE1 Cr 51 M±ES n = 7 | | | |
| --- | --- | --- | --- | --- |
| | Fc | Fab | IgG | RPMI (témoin) |
| 24 μg | 15,5±3,5 >67%* (c) | 46,4±7 >0,01 | 28,0±5,7 >40%* (f) | 47,4±7,4 |
| 50 μg | 12,3±3 >74%* (d) | 45,0±7,5 >0,04 | 24,6±4,5 >48%* (g) | 47,4±7,4 |

\*\*n = 6; \*% d'inhibition

a vs RPMI = p <0,025    e vs RPMI = p <0,05    c vs f = p <0,05
b vs RPMI = p <0,010    f vs RPMI = p <0,05    d vs g = p <0,025
cvs RPMI = p <0,005    g vs RPMI = p <0,02
d vs RPMI = p <0,005

Tableau 3
Activité NK à différents concentrations de fragment Fc, Fab, IgG

| ()/puits Prod | * 6 μg | M±ES % cytotoxicité 12 μg | 24 μg | 100 μg |
| --- | --- | --- | --- | --- |
| Fc | 39,7±8,9 | 40,0±8,9 | 37,7±8,6 | 39,5±9 |
| Fab | 37,5±7,7 | 40,5±8,8 | 38,7±8,9 | 39,2±8,5 |
| IgG | 33,7±8 | 35,0±8 | 34,2±8,2 | 34,7±8,6 |
| RPMI | 37,5±8,4 | 37,5±8,4 | 37,5±8,4 | 37,5±8,4 |

n = 4
\*Rapport 50 cellules effectrices contre 1 cellule-cible.

Tableau 4: CML II
Ratio cellules effectrices – cellules cibles 50:1

| μg/puits | % Cytotoxicité M±ES n = 4 | | | |
| --- | --- | --- | --- | --- |
| | Fc | Fab | IgG | RPMI n = 7 |
| 6 μg | 20,2±1,5 (k) | 21,0±0,5 | 15,0±1,9 (f) | 24,1±2,6 |
| 12 μg | 21,5±1,75 | 20,2±1,1 | 16,7±1,4 (g) | 24,1±2,6 |
| 25 μg | 16,5±2,3 (a) | 14,0±2,9 (d) | 16,7±1,3 (h) | 24,1±2,6 |
| 50 μg | 16,7±3 (b) | 14,7±2,9 (e) | 15,5±2,3 (i) | 24,1±2,6 |
| 100 μg | 15,5±2,7 (c) | 17,7±3,9 | 12,7±2,1 (j) | 24,1±2,6 |

a vs RPMI : p <0,025    e vs RPMI : p <0,025    i vs RPMI : p <0,02
b vs RPMI : p <0,050    f vs RPMI : p <0,005    j vs RPMI : p <0,005
c vs RPMI : p <0,025    g vs RPMI : p <0,005    k vs f : p <0,05
d vs RPMI : p <0,025    h vs RPMI : p <0,005

Cell. eff. (10 J + 2 J R)
  BL* : α1 (2/6 α1/6)    SP* : α1 (7/− 1/7)    GC* : α6 (2/− α 2/6)
  ML* : α3 (2/5 α3/5)    MC* : αND    RP* : ND

Tableau 5: Activité des cellules pré-incubées avec les fragments Fc, Fab et IgG

| Prod | % SUPP. $\overline{X} \pm$ E.S. Fc | Fab | IgG | RPMI (témoin) |
|------|------|------|------|------|
| * conc | | | | |
| 7,5 μg n = 10 | 17 ±5,6 | 18±4,7 | 20,2±3,48 | 15±2,3 |
| 15 μg n = 23 | 31,2±2,9 (a) | 22±3,8 (c) | 43,5±5,7 (e) | 15±2,3 |
| 30 μg n = 23 | 46,2±5,9 (b) | 22±3,4 (d) | 22,3±4,2 (f) | 15±2,3 |

* conc/ml/$10^6$ lymphocytes; a vs RPMI: p<0,005    b vs RPMI: p<0,005
c et d vs RPMI: p NS    e vs RPMI: p<0,005    f vs RPMI: p NS

20

Tableau 5 (suite)

Activité des fragments Fc, Fab, IgG dans l'allosensibilisation.

Test Suppr. A+B+Fc → A+(ABFc)+B

% inhibition $\overline{X} \pm$ ES

| Prod | Fc | Fab | IgG | RPMI |
|------|------|------|------|------|
| conc* | | | | |
| 1,5 μg | 81±2,49 n = 12 | 71 ±5,4 n = 5 | 72 ±8,1 n = 9 | 63,7±3,7 |
| 3 μg | 75±5 | 64 ±7,5 | 76 ±2,8 | 63,7±3,7 |
| 6 μg | 84±1,5 | 66 ±2,98 | 90 ±2,29 | 63,7±3,7 |
| 24 μg | 81±2,76 | 74 ±3,3 | 89,3±2,82 | 63,7±3,7 |
| 100 μg | 84±1,68 | 64,7±5 | 79 ±3,7 | 63,7±3,7 |

Dr SC→ = 3/7α 1/7    DP↓ = 1/7 α5/7    BV↓ = 1/7 α1/–
MB↓ = 5/–α 1/5
Fc 1,5, 6, 24, 100 μg vs RPMI: p<0,005    Fc 3 μg vs RPMI: p = 0,05
IgG 3 μg vs RPMI: p<0,05    IgG 6, 24, 100 μg vs RPMI: p<0,005

* Concentration/200 000 lymphocytes répondeurs

Tableau 5 (suite)

: Test de suppression à différentes concentrations de cellules suppressives

| Prod | D+S→+Fc Fc* | D+(DS↓)←+S↓ % Supp $\overline{X} \pm$ ES Fab* | IgG* | RPMI |
|------|------|------|------|------|
| conc | | | | |
| 50.000→ | 82 ±2,1 | 80 ±1,2 | 74,6±2,1 | 80 ±1,8 |
| 25.000← | 67 ±3,9 | 63,5±2,4 | 53,3±5,3 | 61,5±5,5 |
| 12.000→ | 61,8±4,4 | 51,3±8,4 | 47 ±9,6 | 51,6±10 |
| 6.000↓ | 38 ±4,8 (a) | 30,6±1,5 | 30,8±7,5 | 24 ±5,6 (b) |

Dr: 3/7 α7/–    n = 6    a vs b: p<0,025

* 1,5 μg/200 000 lymphocytes répondeurs

Tableau 5 (suite)
Activité des fragments Fc, Fab, IgG dans le test de suppression de la réponse allogénique I

$$\overline{X} \mp ES \quad + supp$$

| Prod conc | N° exp | RPMI | Fc | Fab | IgG |
|---|---|---|---|---|---|
| 6 µg | 8 | 76,7±3 | 75,8±3,9 | 78,3±3,3 | 70,3±4,62 |
| 12 µg | 13 | 74,4±2,6 | 69,6±3,71 | 74,3±2,61 | 72,7±3,24 |
| 24 µg | 11 | 48,2±4,2 | 64,6±7,4 | 58,3±7,8 | 49,4±4,83 |
| 50 µg | 17 | 54,1±3,9 | 58,4±4,38 | 58,4±4 | 59,2±4,6 |
| 100 µg | 12 | 48,2±4,2 | 59,5±6,56 | 55,1±7,4 | 52,3±5,5 |

Cell. Suppr. SB↓: 5/7 B↓ 1/7 → α1
               BL↓: B 2/6 L↓ 1/6 → α1
               PePi↓: P2 7/3 Pi↓ 7/5 → α5
               6L↓: G 5/7 L 2/5 → α2
               RP↓: R3/5 P 1/7 → α1 et 7

Tableau 6: Production des immunoglobulines totales à partir des différentes sous-populations lymphocytaires.

Lymphocytes Percoll

| Dil. Surn. | $\overline{X}$±ES   ng/ml    n = 4 ind. Fc | | Fab | | IgG n = 5 | RPMI | PWM |
|---|---|---|---|---|---|---|---|
| 1/1 | 2700 | ±1300 | 1060 | ±350 | 100 ±40 | 630 | 1590±887 |
| 1/5 | 256 | ±130 | 350 | ±202 | 58,4±23 | 205 | 2170±1850 |
| 1/25 | 44 | ±29 | 81 | ±42,7 | 47,8±22 | 120 | 135±31 |
| 1/125 | 25,4±15 | | 28,2±24 | | 42,6±20 | 80 | 42±33 |

Lymphocytes NA

| Dil. Surn. | $\overline{X}$±ES   ng/ml    n = 7 ind. Fc | | Fab | | IgG | RPMI | PWM | |
|---|---|---|---|---|---|---|---|---|
| 1/1 | 520±160 | | 229 | ±110 | 53±18 | 200 | 1210 | ±1000 |
| 1/5 | 123±63 | | 110 | ±47 | 50±20 | 60 | 455 | ±253 |
| 1/25 | 67±24 | | 28,1±11,5 | | 44±19 | 65 | 11 | ±5 |
| 1/125 | 13±11 | | 29,5±10 | | 35±16 | 60 | 9,3±2,7 | |

Tableau 6 (suite)
Lymphocytes de sang périphérique

| Dil. Surn. | $\overline{X}$±ES   ng/ml    n = 4 ind. Fc | | Fab | | Ig | RPMI | PWM |
|---|---|---|---|---|---|---|---|
| 1/1 | 750 | ±330 | 207 | ±30 | 115 ±32 | 485 | 2320±183 |
| 1/5 | 132 | ±31 | 96,7±15 | | 73,2±10 | 185 | 390±197 |
| 1/25 | 58,2±19 | | 65,7±14 | | 68,5±16 | 120 | 74±21 |
| 1/125 | 37,2±10 | | 44 | ±18 | 51,7±16,9 | 60 | 43±16 |

**Revendications pour les Etats contractants: BE, CH, DE, FR, GB, IT, LI, LU, NL, SE**

1. Médicament immunomodulateur à usage humain, caractérisé par le fait que son principe actif est constitué par des fragments Fc d'IgG humaines, ou par des analogues desdits fragments, et qu'il est exempt d'IgG intactes et/ou de fragments Fab ou qu'il n'en contient pas plus de 2% en poids par rapport au poids total (Fc + Fab + IgG), étant entendu que lesdits analogues des fragments Fc d'IgG humaines sont tous fragments ou sous-fragments de Fc et tous peptides synthétiques ou semi-synthétiques contenant des séquences peptidiques présentes sur le fragment Fc, ainsi que tous dérivés de ces fragments, sous-fragments ou peptides, à condition que ces fragments, sous-fragments, peptides ou dérivés satisfassent aux conditions suivantes:

– ils ont des propriétés d'activateurs de la différenciation des cellules suppressives;

– ils ont des propriétés d'inhibiteurs de la cytotoxicité anticorps-dépendante;

– ils n'ont pas de propriétés d'activateurs des cellules NK.

2. Médicament selon la revendication 1, caractérisé par le fait qu'il ne contient pas plus de 1% en poids de fragments Fab et/ou d'IgG intactes.

3. Médicament selon la revendication 1, caractérisé par le fait qu'il est exempt de fragments Fab et/ou d'IgG intactes.

4. Médicament selon l'une quelconque des revendications précédentes, caractérisé par le fait que le principe actif est constitué par des fragments Fc.

5. Utilisation du fragment Fc des IgG humaines ou de ses analogues, tels qu'ils sont définis dans la revendication 1, dans la préparation d'un médicament immunomodulateur à usage humain.

6. Utilisation selon la revendication 5, caractérisé par le fait que ledit médicament immunomodulateur est destiné à traiter les maladies auto-immunes.

7. Utilisation selon la revendication 5, caractérisé par le fait que ledit médicament immunomodulateur est destiné à prévenir les réactions de rejet ou d'en diminuer les effets, chez les malades devant subir ou ayant subi une greffe d'organe.

**Revendications pour l'Etat contractant: AT**

1. Utilisation comme principe actif, dans la préparation d'un médicament immunomodulateur à usage humain, de fragments Fc d'IgG humains ou d'analogues desdits fragments, étant entendu que lesdits analogues des fragments Fc d'IgG humaines sont tous fragments ou sous-fragments de Fc et tous peptides synthétiques ou semi-synthétiques contenant des séquences peptidiques présentes sur le fragment Fc, ainsi que tous dérivés de ces fragments, sous-fragments ou peptides, à condition que ces fragments, sous-fragments, peptides ou dérivés satisfassent aux conditions suivantes:

– ils ont des propriétés d'activateurs de la différenciation des cellules suppressives;

– ils ont des propriétés d'inhibiteurs de la cytotoxicité anticorps-dépendante;

– ils n'ont pas de propriétés d'activateurs des cellules NK; et étant entendu que ledit médicament est exempt d'IgG intactes et/ou de fragments Fab ou qu'il n'en contient pas plus de 2% en poids par rapport au poids total (Fc + Fab + IgG).

2. Utilisation selon la revendication 1, caractérisée par le fait que ledit médicament ne contient pas plus de 1% en poids de fragments Fab et/ou d'IgG intactes.

3. Utilisation selon la revendication 1, caractérisée par le fait que ledit médicament est exempt de fragments Fab et/ou d'IgG intactes.

4. Utilisation selon l'une quelconque des revendications précédentes, caractérisée par le fait que ledit principe actif est constitué par des fragments Fc.

5. Utilisation selon l'une quelconque des revendications précédentes, caractérisé par le fait que ledit médicament immunomodulateur est destiné à traiter les maladies auto-immunes.

6. Utilisation selon l'une quelconque des revendications 1 à 4, caractérisée par le fait que ledit médicament immunomodulateur est destiné à prévenir les réactions de rejet ou d'en diminuer les effets, chez les malades devant subir ou ayant subi une greffe d'organe.

**Claims for the contracting States: BE, CH, DE, FR, GB, IT, LI, LU, NL, SE**

1. Immunomodulatory drug for human use, characterized in that its active principle consists of Fc fragments of human IgG's, or of analogues of the said fragments, and in that it is free from intact IgG's and/or from Fab fragments or in that it contains not more than 2% by weight thereof relative to the total weight (Fc + Fab + IgG), it being understood that the said analogues of Fc fragments of human IgG's are all fragments or subfragments of Fc and all synthetic or semi-synthetic peptides containing peptide sequences present on the Fc fragment, as well as all derivatives of these fragments, subfragments or peptides, on condition that these fragments, subfragments, peptides or derivatives satisfy the following conditions:

– they have properties of activators of the differentiation of suppressor cells;

– they have properties of inhibitors of antibody-dependent cytotoxicity;

– they do not have properties of activators of NK cells.

2. Drug according to Claim 1, characterized in that it does not contain more than 1% by weight of Fab fragments and/or of intact IgG's.

3. Drug according to Claim 1, characterized in that it is free from Fab fragments and/or from intact IgG's.

4. Drug according to any one of the preceding claims, characterized in that the active principle consists of Fc fragments.

5. Use of the Fc fragment of human IgG's or its analogues, as are defined in Claim 1, in the preparation of an immunomodulatory drug for human use.

6. Use according to Claim 5, characterized in that the said immunomodulatory drug is intended for treating autoimmune disease.

7. Use according to Claim 5, characterized in that the said immunomodulatory drug is intended for preventing rejection reactions, or reducing the effects thereof, in patients who have to undergo or have undergone organ transplantation.

**Claims for the Contracting State: AT**

1. Use as active principle, in the preparation of an immunomodulatory drug for human use, of Fc fragments of human IgG's, or of analogues of the said fragments, it being understood that the said analogues of Fc fragments of human IgG's are all fragments or subfragments of Fc and all synthetic or semi-synthetic peptides containing peptide sequences present on the Fc fragment, as well as all derivatives of these fragments, subfragments or peptides, on condition that these fragments, subfragments, peptides or derivatives satisfy the following conditions:
   — they have properties of activators of the differentiation of suppressor cells;
   — they have properties of inhibitors of antibody-dependent cytotoxicity;
   — they do not have properties of activators of NK cells; and it being understood that the said drug is free from intact IgG's and/or from Fab fragments or that it contains not more than 2% by weight thereof relative to the total weigt (Fc + Fab + IgG).

2. Use according to claims 1, characterized in that the said drug does no contain more than 1% by weight of Fab fragments and/or of intact IgG's.

3. Use according to claim 1, characterized in that the said drug is free from Fab fragments and/ or from intact IgG's.

4. Use according to claim 1, characterized in that the said drug consists of Fc fragments.

5. Use according to any one of the preceding claims, characterized in that the said immunomodulatory drug is intended for treating autoimmune diseases.

6. Use according to any one of Claims 1 to 4, characterized in that the said immunomodulatory drug is intended for preventing rejection reactions, or reducing the effects thereof, in patients who have to undergo or have undergone organ transplantation.

**Patentansprüche für die Vertragsstaaten: BE, CH, DE, FR, GB, IT, LI, LU, NL, SE**

1. Immunomodulierendes Arzneimittel für den Gebrauch bei Menschen, dadurch gekennzeichnet, daß sein Wirkstoff aus Fragmenten Fc von menschlichem Immunglobulin IgG oder Analogen dieser Fragmente zusammengesetzt ist und daß es frei ist von intakten IgG und/oder von Fragmenten Fab oder daß es davon nicht mehr als 2 Gew.-

%, bezogen auf das Gesamtgewicht (Fc + Fab + IgG), enthält, wobei die Analogen der Fragmente Fc von menschlichem IgG alle Fragmente oder Unterfragmente von Fc und alle synthetischen Peptide oder halbsynthetischen Peptide, die Peptidsequenzen enthalten, die im Fragment Fc vorkommen, sowie alle Derivate solcher Fragmente, Unterfragmente oder Peptide, sind, wobei diese Fragmente, Unterfragmente, Peptide oder Derivate folgende Bedingungen erfüllen:
   daß sie die Eigenschaft von Aktivatoren der Differenzierung von suppressiven Zellen haben;
   daß sie die Eigenschaften von Inhibitoren der antikörperabhängigen Cytotoxizität haben;
   daß sie nicht die Eigenschaften von Aktivatoren der Zellen NK aufweisen.

2. Arzneimittel nach Anspruch 1, dadurch gekennzeichnet, daß es nicht mehr als 1 Gew.-% von Fragmenten Fab und/oder intakten IgG enthält.

3. Arzneimittel nach Anspruch 3, dadurch gekennzeichnet, daß es frei ist von Fragmenten Fab und/oder intakten IgG.

4. Arzneimittel nach jedem der vorhergehenden Ansprüche, dadurch gekennzeichnet, daß der Wirkstoff aus Fragmenten Fc besteht.

5. Verwendung eines Fragments Fc von menschlichem IgG oder dessen Analogen, wie sie in Anspruch 1 definiert sind, zur Herstellung eines immunmodulierenden Arzneimittels.

6. Verwendung nach Anspruch 5, dadurch gekennzeichnet, daß das immunmodulierende Arzneimittel dazu bestimmt ist, Autoimmunkrankheiten zu behandeln.

7. Verwendung nach Anspruch 5, dadurch gekennzeichnet, daß das immunmodulierende Arzneimittel dazu bestimmt ist, Abstoßungsreaktionen vorzubeugen, oder solche Wirkungen zu vermindern, die bei Krankheiten während oder nach einer Organverpflanzung auftreten bzw. aufgetreten sind.

**Patentansprüche für den Vertragsstaat: AT**

1. Verwendung von Fragmenten Fc von menschlichem Immunglobulin IgG oder Analogen dieser Fragmente, wobei die Analogen der Fragmente Fc alle Fragmente oder Unterfragmente von Fc und alle synthetischen Peptide oder halbsynthetischen Peptide, die Peptidsequenzen enthalten, die im Fragment Fc vorkommen sind, sowie alle Derivate solcher Fragmente, Unterfragmente oder Peptide, sind, wobei diese Fragmente, Unterfragmente, Peptide oder Derivate folgende Bedingungen erfüllen:
   daß sie die Eigenschaft von Aktivatoren der Differenzierung von suppressiven Zellen haben,
   daß sie die Eigenschaften von Inhibitoren der antikörperabhängigen Cytotoxizität haben,
   daß sie nicht die Eigenschaften von Aktivatoren der Zellen NK aufweisen,
   als Wirkstoff zur Herstellung eines immunomodulierenden Arzneimittels für den Gebrauch bei Menschen, und wobei diese Arzneimittel frei von intakten IgG und/oder von Fragmenten Fab ist, oder daß es davon nicht mehr als 2 Gew.-%, bezogen auf das Gesamtgewicht (Fc + Fab + IgG), enthält.

2. Verwendung nach Anspruch 1, dadurch gekennzeichnet, daß diese Arzneimittel nicht mehr als 1 Gew.-% von Fragmenten Fab und/oder intakten IgG enthält.

3. Verwendung nach Anspruch 1, dadurch gekennzeichnet, daß diese Arzneimittel frei von Fragmenten Fab und/oder intakten IgG ist.

4. Verwendung nach Anspruch 1, dadurch gekennzeichnet, daß der Wirkstoff aus Fragmenten Fc besteht.

5. Verwendung nach jedem der vorhergehenden Ansprüche, dadurch gekennzeichnet, daß das immunmodulierende Arzneimittel dazu bestimmt ist, Autoimmunkrankheiten zu behandeln.

6. Verwendung nach einem der Patentansprüche 1 bis 4, dadurch gekennzeichnet, daß das immunmodulierende Arzneimittel dazu bestimmt ist, Abstoßungsreaktionen vorzubeugen, oder solche Wirkungen zu vermindern, die bei Krankheiten während oder nach einer Organverpflanzung auftreten bzw. aufgetreten sind.